# EUROPEAN PATENT APPLICATION

(11) **EP 3 786 636 A1**
(43) Date of publication of application: **03.03.2021**
(21) Application number: 19807736.4
(22) Date of filing: 21.05.2019
(51) Int. Cl.: G01N 31/00, G01N 27/62

(54) **ELEMENT ANALYSIS DEVICE AND ELEMENT ANALYSIS METHOD**

(30) Priority: 25.05.2018 JP 2018100272; 19.07.2018 JP 2018135772
(71) Applicant: HORIBA, Ltd., Kyoto 601-8510 (JP); HORIBA STEC, Co., Ltd., Kyoto-shi, Kyoto 601-8116 (JP)
(72) Inventor: INOUE, Takahito, Kyoto-shi, Kyoto 601-8510 (JP); UCHIHARA, Hiroshi, Kyoto-shi, Kyoto 601-8510 (JP); SASAI, Kohei, Kyoto-shi, Kyoto 601-8116 (JP); IKEYAMA, Toshihiro, Kyoto-shi, Kyoto 601-8116 (JP); SEKI, Hiroyuki, Kyoto-shi, Kyoto 601-8116 (JP)
(74) Representative: Isarpatent
(86) International application number: PCT/JP2019/020032
(87) International publication number: WO 2019/225573

(57) **Abstract**

An element analysis device is provided, which can quantitatively analyze an H element contained in a sample gas with high accuracy. The element analysis device is provided with a heating furnace for heating a crucible having a sample contained therein while a carrier gas is introduced into the heating furnace to vaporize at least a part of the sample to generate a sample gas containing the H element and then deriving the sample gas with the carrier gas as a mixed gas and a mass spectrometer for quantitatively analyzing at least one element contained in the sample gas in the mixed gas that has been derived from the heating furnace, wherein the mass spectrometer quantitatively analyzes the H element contained as an H₂O component in the mixed gas as the H element contained in the sample gas.

## Description

### Field of the art

This invention relates to an element analysis device and an element analysis method.

### Background art

A conventional element analysis device comprises, as described in the patent document 1, a heating furnace that heats a crucible into which a sample is put while a carrier gas is introduced into the heating furnace, generates a sample gas by vaporizing at least a part of the sample and derives a mixed gas as a mixture of the sample gas and the carrier gas and a quantitative analyzer that quantitatively analyzes at least one element contained in the sample gas in the mixed gas derived from the heating furnace.

In case that the conventional element analysis device quantitatively analyzes a trace element such as H element contained in a sample such as, for example, steel, the conventional element analysis device conducts a quantitative analysis by generating the sample gas containing an H element as an H2 component, and introducing the sample gas as the mixed gas composed of the carrier gas into a mass spectrometer.

More specifically, the conventional element analysis device quantitatively analyzes the H element contained as the H2 component in the mixed gas as the H element contained in the sample gas. However, in this case, it is not possible for the mass spectrometer to sensitively detect the H2 component whose mass number is small so that there is a problem that it is not possible to conduct the quantitative analysis on the H element contained in the sample gas with high accuracy.

### Prior art documents

### [Patent document]

[Patent document 1] Japanese Unexamined Patent Application Publication No. 2000 - 2699

### Disclosure of the invention

### Problems to be solved by the invention

A main object of this invention is to obtain an element analysis device that can quantitatively analyze the H element contained in the sample gas with high accuracy.

### Means to solve the problems

More specifically, an element analysis device in accordance with this invention comprises a heating furnace that heats a crucible having a sample contained therein while a carrier gas is introduced into the crucible to generate a sample gas containing an H element by vaporizing at least a part of the sample and derives the sample gas as a mixed gas as being a mixture of the sample gas and the carrier gas, and a mass spectrometer that quantitatively analyzes at least one element contained in the sample gas in the mixed gas that is derived from the heating furnace, and is characterized by that the mass spectrometer quantitatively analyzes the H element contained as an H₂O component in the mixed gas regarded as the H element contained in the sample gas.

In accordance with this arrangement, in case that the H element contained in the sample gas is to be quantitatively analyzed, since the H element is introduced into the mass spectrometer as the H₂O component whose mass number is big, it becomes possible for the mass spectrometer to sensitively detect the H element. As a result of this, it becomes possible to quantitatively analyze the H element contained in the sample gas with high accuracy.

Concretely, the element analysis device may further comprise an oxidation part arranged between the heating furnace and the mass spectrometer, and may be so configured that the heating furnace generates the sample gas containing the H element as an H containing component and derives the mixed gas containing the H containing component, the oxidation part oxidizes the H containing component in the mixed gas to generate the H₂O component, and the mass spectrometer quantitatively analyzes the H element contained as the H₂O component in the oxidized mixed gas regarded as the H element contained in the sample gas.

Furthermore, for example, in case of generating the sample gas containing both the H element as the H₂ component and an N element and an O element as being the element other than the H element as an N₂ component and a CO component respectively, it is necessary not only to generate the H₂O component by oxidizing the H2 component in the mixed gas by the oxidation part but also to generate a CO2 component by oxidizing the CO component in the mixed gas. The reason is that the mass number of the N2 component and that of the CO component are the same, namely 28, then the time zone when the peak indicating each component detected by the mass spectrometer overlaps each other when the N₂ component and the CO component are introduced into the mass spectrometer as it is so that it becomes difficult to separate the peak.

Then, the element analysis device may be so configured that the heating furnace generates the sample gas that contains the H element as the H containing component and that contains a plurality of elements other than the H element as other element containing components wherein each element differs, and derives the mixed gas containing the H containing component and the plurality of the other element containing components, the oxidation part generates the H₂O component by oxidizing the H containing component in the mixed gas and oxidizes at least one of the two other element containing components wherein a mass number difference between the two other elements of the two other element containing components in the mixed gas is three or less, and the mass spectrometer quantitatively analyzes the H element contained as the H₂O component in the oxidized mixed gas as the H element contained in the sample gas and quantitatively analyzes the other element other than the H element contained as the other element containing component in the oxidized mixed gas regarded as the other element contained in the sample gas.

In accordance with this arrangement, since at least one of the two other element containing components whose mass number difference of the two other elements is three or less is oxidized by the oxidation part, the mass number difference between the two other elements of the two other element containing components after being oxidized becomes bigger than three. In other words, the mass number difference between the two other elements of the two other element containing components having the nearest mass number each other and contained in the oxidized mixed gas becomes three or more. As a result of this, a time zone when the peak, indicating the oxidized two other element containing components detected by the mass spectrometer, appears is deviated.

In addition, it is preferable to use a He gas as the carrier gas. There is a case that an Ar gas is used as the carrier gas of this kind of the element analysis device. However, since a molecular diameter of an Ar atom is relatively big, the component containing the element to be analyzed is prevented from being ionized by the mass spectrometer. Meanwhile, since the molecular diameter of a He atom is relatively small, the component containing the element to be analyzed is not prevented from being ionized by the mass spectrometer. Furthermore, since the He is longer in a mean free process compared with the Ar, it is possible to set the chamber pressure of the He gas higher than that of the Ar gas. Accordingly, the component containing the element to be analyzed is effectively ionized by the mass spectrometer by using the He gas as the carrier gas. As a result of this, it becomes possible to detect more ions of the component containing the element to be analyzed by the mass spectrometer, resulting in being able to conduct the quantitative analysis on the H element contained in the sample gas with high accuracy.

In addition, in case of using a quadrupole mass spectrometer as the mass spectrometer, it is preferable that the quadrupole mass spectrometer comprises an operation part, wherein the operation part comprises a reference data preparing part that prepares a reference data indicating a chronological change of a signal intensity by applying heat to the crucible by the heating furnace in a state wherein no sample is fed into the crucible while introducing the carrier gas into the heating furnace, and by introducing the carrier gas derived from the heating furnace into the quadrupole mass spectrometer, a measurement data preparing part that prepares a measurement data indicating the chronological change of the signal intensity by applying heat to the crucible by the heating furnace in a state wherein the sample is fed into the crucible while the carrier gas is introduced into the heating furnace, and by introducing the mixed gas derived from the heating furnace into the quadrupole mass spectrometer, a peak time zone specifying part that specifies a peak time zone in the measurement data when a peak of the signal intensity corresponding to the component containing the element contained in the sample gas in the mixed gas is detected, a correction value calculating part that calculates, as a correction value, an area of a part surrounded by the reference data and a straight line connecting a starting point and an ending point in a predetermined time zone that is determined so as not to include time when the signal intensity in the reference data become the maximum value and the minimum value and to include the peak time zone, and an analyzing part that quantitatively analyzes the element contained in the sample gas based on the measurement data and the correction value.

In case that the element contained in the sample gas is quantitatively analyzed by the quadrupole mass spectrometer, the element contained in the sample gas is quantitatively analyzed by correcting the value obtained based on the measurement data by the correcting value obtained based on the reference data. In this case, if adopting the area of a part surrounded by the reference data and the straight line connecting the starting point and the ending point in the predetermined time zone that is determined so as not to include time when the signal intensity in the reference data becomes the maximum value and the minimum value and to include the peak time zone as the correction value, the lower limit of being able to conduct the quantitative analysis is extended so that it becomes possible to conduct the quantitative analysis in a wider range.

In addition, in case of conducting the quantitative analysis on the element contained in the sample gas by means of the element analysis device, the pressure in the chamber to which the mass spectrometer is connected is kept near vacuum. The present claimed inventor found that there exists a pressure that is detected sensitively for each element contained in the sample gas. Then, the element analysis device may further comprise a chamber that is arranged in a downstream side of the heating furnace and to which the mass spectrometer is connected, and a pressure adjusting mechanism that can adjust pressure in the chamber according to the element to be quantitatively analyzed by the mass spectrometer.

More concretely, the pressure adjusting mechanism may comprise a valve arranged between the heating furnace and the chamber and an exhaust pump arranged in the downstream side of the chamber, and the pressure in the chamber is adjusted based on an opening of the valve. Furthermore, the element analysis device may further comprise a pressure sensor that measures the pressure in the chamber, and the pressure adjusting mechanism may comprise a pressure control part that controls the pressure value measured by the pressure sensor to approach a predetermined set pressure according to the element to be quantitatively analyzed by the mass spectrometer.

For example, the set pressure in case of conducting the quantitative analysis on the H₂O component containing the H element by means of the mass spectrometer is preferably 1.0 Pa or more, and more preferably 1.0 Pa or more and 3.5 Pa or less.

In addition, an element analysis method in accordance with this invention is an element analysis methods for quantitatively analyzing an element contained in a sample gas generated by vaporizing a sample, and the method is applying heat to a crucible having the sample contained therein while introducing a carrier gas to generate the sample gas that contains an H element by vaporizing at least a part of the sample, introducing the H element in a mixed gas comprising the sample gas and the carrier gas derived from the heating furnace into a mass spectrometer as an H₂O component, and quantitatively analyzing the H element contained as the H₂O component in the mixed gas by the use of the mass spectrometer.

In this case, the mass spectrometer may be a quadrupole mass spectrometer, and the element analysis method may prepare a reference data indicating a chronological change of a signal intensity by applying heat to the crucible by the heating furnace in a state wherein no sample is fed into the crucible while the carrier gas is introduced into the heating furnace, and by introducing the carrier gas derived from the heating furnace into the quadrupole mass spectrometer, prepare a measurement data indicating the chronological change of the signal intensity by applying heat by the heating furnace to the crucible in a state wherein the sample is fed into the crucible while the carrier gas is introduced into the heating furnace, and by introducing the mixed gas derived from the heating furnace into the quadrupole mass spectrometer, specify a peak time zone in the measurement data when a peak of the signal intensity corresponding to the component containing the element contained in the sample gas in the mixed gas is detected, calculate an area of a part surrounded by the reference data and a straight line connecting a starting point and an ending point in a predetermined time zone that is determined so as not to include time when the signal intensity in the reference data becomes the maximum value and the minimum value and so as to include the peak time zone as a correction value, and quantitatively analyze the element contained in the sample gas based on the measurement data and the correction value.

### Effect of the invention

In accordance with the element analysis device having the above arrangement, it is possible to quantitatively analyze an H element contained in a sample gas with high accuracy.

### Brief description of the drawings

[Fig. 1] A pattern view showing an overall structure of an element analysis device in accordance with a first embodiment.
[Fig. 2] A block diagram showing an operation part of a quadrupole mass spectrometer of the element analysis device in accordance with this embodiment.
[Fig. 3] A graph showing a reference data and a measurement data obtained by the analysis operation of the element analysis device in accordance with this embodiment.
[Fig. 4] A graph showing an enlarged area around a peak of the measurement data obtained by the analysis operation of the element analysis device in accordance with this embodiment
[Fig. 5] A graph showing a relationship between a chamber pressure and a signal intensity of the element analysis device in accordance with this embodiment.
[Fig. 6] A schematic diagram showing an overall structure of an elemental analysis device in accordance with another embodiment.

### Explanation of codes

- 100: element analysis device
- 10: heating furnace
- 11: crucible
- 20: carrier gas supplier
- 30: chamber
- 40: quadrupole mass spectrometer
- 50: pressure adjusting mechanism
- 51: pressure adjusting valve
- 52: exhaust mechanism
- 80: oxidation part

### Best modes of embodying the invention

An element analysis device in accordance with this invention will be explained with reference to drawings.

The element analysis device of this embodiment applies heat to and melts a sample such as steel or ceramics put into a crucible and extracts and quantitatively analyzes an element contained in a sample gas that is generated while heat is applied thereto.

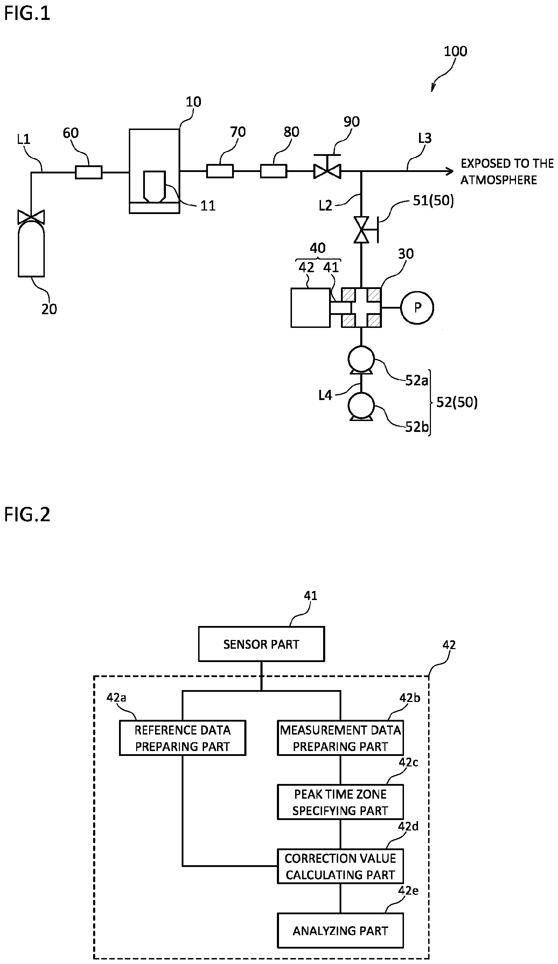

<First embodiment The element analysis device 100 in accordance with this embodiment comprises, as shown in Fig. 1, a heating furnace 10, an upstream line L1 extending upstream from the heating furnace 10, a carrier gas supplier 20 connected to a starting end of the upstream line L1, a downstream line L2 extending downstream from the heating furnace 10, a chamber 30 connected to a terminal end of the downstream line L2, a quadrupole mass spectrometer 40 connected to the chamber 30 and a pressure adjusting mechanism 50 for adjusting a pressure in the chamber 30.

The heating furnace 10 is a so-called impulse furnace and houses a crucible 11 into which a sample is put. The heating furnace 10 passes an impulse current through the crucible 11 to generate the Joule heat, thereby producing the sample gas by evaporating at least a part of the sample put into the crucible 11. A graphite crucible may be used as the crucible 11.

The upstream line L1 introduces a carrier gas supplied from the carrier gas supplier 20 into the heating furnace 10. In addition, a purifier 60 for purifying the carrier gas is arranged in the middle of the upstream line L1. An inactive gas is used as the carrier gas, and the He gas is especially preferable.

The downstream line L2 introduces a mixed gas comprising the sample gas and the carrier gas derived from the heating furnace 10 into the chamber 30. A first exhaust line L3 that bifurcates and extends from the middle of the downstream line L2 is connected to the downstream lime L2. A terminal end of the first exhaust line L3 is exposed to the atmosphere.

The chamber 30 comprises four ports. Each of the downstream line L3, the quadrupole mass spectrometer 40, a pressure sensor (P) for measuring the pressure in the chamber 30 and a second exhaust line L4 is connected to each of the four ports respectively.

The quadrupole mass spectrometer 40 quantitatively analyzes the element (hereinafter also called as an element to be analyzed) contained in the sample gas in the mixed gas. Concretely, the quadrupole mass spectrometer 40 comprises a sensor part 41 that ionizes the component that contains the element to be analyzed and that detects the ion and an operation part 42 that quantitatively analyzes the element to be analyzed by referring a chronological change of a signal intensity detected by the sensor part 41. The signal intensity is, for example, a current intensity or an electromagnetic intensity. The quadrupole mass spectrometer 40 is connected to the chamber 30 in a state wherein the sensor part 41 is inserted into the port.

The operation part 42 is a so-called computer comprising a CPU, a memory, an A/D convertor, and a D/A convertor, and so structured to produce a function of conducting the quantitative analysis on the element to be analyzed by executing programs stored in the memory.

Concretely, the operation part 42 comprises a reference data preparing part 42a to prepare a reference data, a measurement data preparing part 42b to prepare a measurement data, a peak time zone specifying part 42c to specify a peak time zone in the measurement data, a correction value calculating part 42d to calculate a correction value based on the reference data and the peak time zone and an analysis part 42e to conduct the quantitative analysis on the element to be analyzed based on the reference data and the correction value.

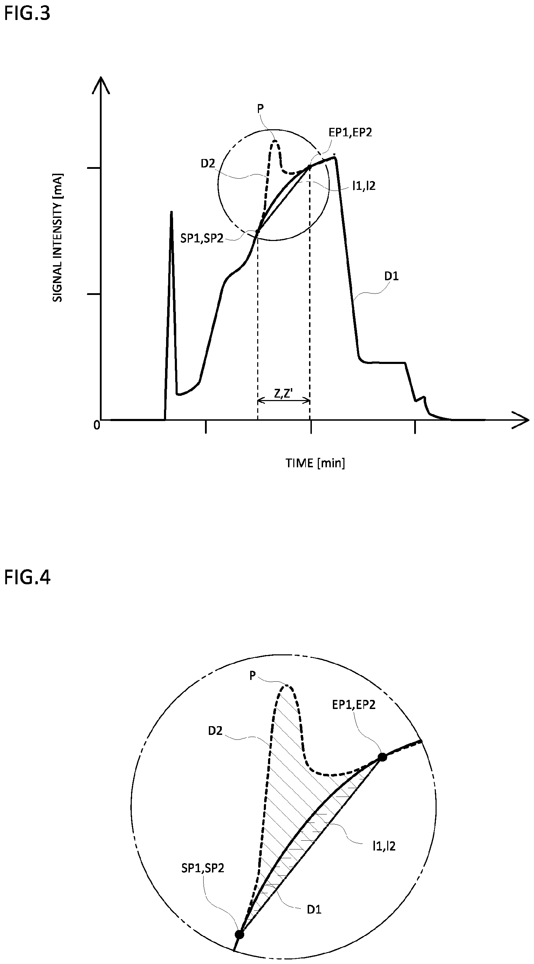

The reference data preparing part 42a prepares the reference data through the following process. More in detail, first, the crucible 11 is heated by the heating furnace 10 in a state wherein no sample is put into the crucible 11 while the carrier gas is introduced into the heating furnace 10 from the upstream line L1. Next, the carrier gas derived from the heating furnace 10 to the downstream line L2 is introduced into the quadrupole mass spectrometer 40. Then, the reference data preparing part 42a prepares a reference data D1 (data shown by a solid line in Fig. 3) that plots the chronological change of the signal intensity detected when the carrier gas is introduced into the quadrupole mass spectrometer 40.

The measurement data preparing part 42b prepares the measurement data through the followings processes. More in detail, first, the crucible 11 is heated by the heating furnace 10 in a state wherein the sample is put into the crucible 11 while the carrier gas is introduced into the heating furnace 10 from the upstream line L1. Next, the mixed gas derived from the heating furnace 10 to the downstream line L2 is introduced into the quadrupole mass spectrometer 40. Then, the measurement data preparing part 42b prepares a measurement data D2 (data shown by a dotted line in Fig. 3) that plots the chronological change of the signal intensity detected when the mixed gas is introduced into the quadrupole mass spectrometer 40.

The peak time zone specifying part 42c specifies the peak time zone (Z) when the peak (P) of the signal intensity corresponding to the component containing the element to be analyzed in the measurement data D2. For example, as shown in Fig. 3 and Fig. 4, the peak (P) in the measurement data D2 appears to project in a convex state with respect to the reference data D1. Then, with the time when the signal intensity starts to rise toward the peak (P) regarded as a starting point (SP) and the time when the signal intensity after the peak (P) regarded as an ending point (EP), the peak time zone (Z) is defined as a time zone between the starting point (SP) and the ending point (EP). Concretely, the starting point (SP) of the peak time zone (Z) is a time of a transition point when a state wherein a gradient of a tangential line of the measurement data D2 coincides with the gradient of the tangential line passing the reference data D1 changes to a state wherein the gradient of the tangential line of the measurement data D2 does not coincide with the gradient of the tangential line passing the reference data D1 at a time of a rising phase of the signal intensity before the peak (P), and the ending point (EP) of the peak time zone (Z) is a time of a transition point when a state wherein a gradient of a tangential line of the measurement data D2 does not coincide with the gradient of the tangential line passing the reference data D1 changes to a state wherein the gradient of the tangential line of the measurement data D2 coincides with the gradient of the tangential line passing the reference data D1 at a time after the peak (P). Therefore, the peak time zone (Z) is the time zone before and after including the time when the signal intensity at peak (P) becomes the maximum (the maximum value).

The correction value calculating part 42d calculates, as a correction value, an area of a part (a part shown by a dotted line diagonal line in Fig. 4) surrounded by the reference data D1 and a straight line 11 connecting a starting point SP1 and an ending point EP1 in a predetermined time zone (Z') which is determined not to include the time when the signal intensity in the reference data D1 becomes the maximum value and the minimum value and to include the peak time zone (Z). In this embodiment, the predetermined time zone (Z') is set to coincide with the peak time zone (Z), however, the predetermined time period (Z') may be set to include the peak time zone (Z).

The analysis part 42e is to conduct the quantitative analysis on the element to be analyzed based on the measurement data D2 and the correction value. Concretely, the analysis part 42e calculates an area of a part (a part shown by the solid line diagonal line in Fig. 4) surrounded by the measurement data D2 and a straight line I2 connecting a starting point SP2 and an ending point EP2 in the predetermined time zone (Z) in the measurement data D2, and calculates an ionic concentration of the component containing the element to be analyzed by subtracting the correction value from the calculated value. Then, the analysis part 42e calculates the concentration of the component containing the element to be analyzed and the amount of the element to be analyzed by referring to known values of the concentration of the ion of the component containing the element to be analyzed and the mass number of the element contained in the ion. In this embodiment, since the starting point SP1 and the ending point EP1 of the predetermined time zone (Z') in the reference data D1 and the starting point SP2 and the ending point EP2 of the predetermined time zone (Z') in the measurement data D2 coincide with each other, the straight line 11 and the straight line 12 become the same straight line. There may be a case wherein the starting point SP1 and the ending point EP1 do not coincide with the starting point SP2 and the ending point EP2 respectively depending on a shape of the reference data D1 or the measurement data D2 or a setting of the predetermined time zone (Z'). In this case, of course, the straight line 11 and the straight line 12 are not the same line.

The pressure adjusting mechanism 50 comprises a pressure control valve 51 arranged in the downstream line L2 and downstream of a junction between the first exhaust line L3 and the downstream line L2, an exhaust mechanism 52 arranged in the second exhaust line L4, and a pressure control part (not shown in drawings) for controlling the pressure in the chamber 30 by controlling the opening of the pressure control valve 51. A needle valve can be used as the pressure control valve 51. In addition, a turbo pump and a dry pump can be used as the exhaust mechanism 52. The exhaust mechanism 52 of this embodiment is so configured that the turbo pump 52a arranged in the upstream side and the dry pump arranged in the downstream side are arranged in series.

The pressure control part is a so-called computer comprising a CPU, a memory, an A/D converter and a D/A converter, and produces a function to control the opening of the pressure adjusting valve 51 so as to make the pressure value of the pressure sensor (P) close to a predetermined set pressure by executing programs stored in the memory.

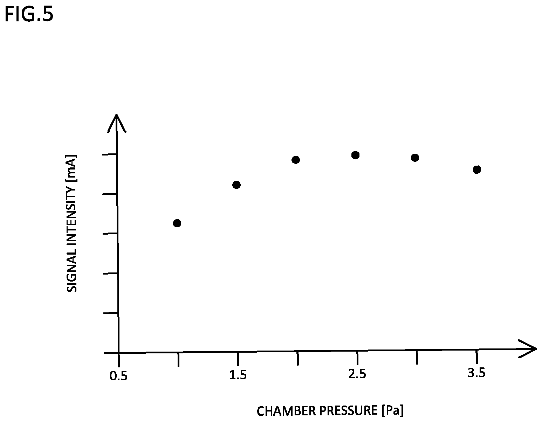

The set pressure is a pressure predetermined for each component containing the element to be analyzed. More specifically, in case that the signal intensity is measured by introducing the H₂O component containing the H element as being the element to be analyzed into the quadrupole mass spectrometer 40, if conditions are the same except for the pressure in the chamber 30, it becomes clear that the signal intensity increases when the pressure in the chamber 30 is 1.5 Pa or higher, as shown in Fig. 5. This indicates that the sensitivity of the quadrupole mass spectrometer 40 is improved by keeping the pressure in the chamber 30 at 1.5 Pa or higher when measuring the signal intensity by introducing the H₂O component into the quadrupole mass spectrometer 40. Therefore, the pressure is verified wherein the sensitivity is improved for each component containing the element to be analyzed in advance, and the pressure is set as the set pressure. The set pressure can be stored in the memory arranged in the pressure control part and can be switched as needed.

Next, other equipment to be installed in the downstream line L2 will be explained.

A dust filter 70, an oxidation part 80 and an exhaust volume adjusting valve 90 are arranged in this order from the upstream side towards the downstream side in the downstream line L2 in the upstream side of the junction between the downstream line L2 and the first exhaust line L3.

The dust filter 70 is for removing dust such as soot from the mixed gas derived from the heating furnace 10.

The oxidation part 80 is for oxidizing the components contained in the mixed gas. Concretely, in case that CO or H2 is contained in the sample gas generated in the heating furnace 10, the oxidation part 80 changes CO or H2 into CO2 or H₂O by oxidizing CO or H2. More specifically, a high temperature oxidizing agent such as copper oxide may be used as the oxidation part 80.

The exhaust volume adjusting valve 90 adjusts the exhaust volume of the mixed gas exhausted from the first exhaust line L3. A needle valve can be used as the exhaust volume adjusting valve 90. The exhaust volume adjusting valve 90 may be arranged in the first exhaust line L3.

In addition, a heater (not shown in the figure) for heating a portion from the oxidation part 80 to the chamber 30 is arranged in the downstream line L2. With this arrangement, it is possible for the H₂O component flowing in the downstream line L2 from the oxidation part 80 to the chamber 30 to prevent from staying in the downstream line L2 in a form of water droplets.

Next, the operation of the case in which the elements contained in the sample gas are quantitatively analyzed using the elemental analysis device 100 will be explained. In explaining the operation, explained is a case that a sample gas containing the H2 component, the CO component and the N2 component are generated by vaporizing the sample gas by the use of the He gas as the carrier gas, and the O element, the N element and the H element contained in the sample gas are quantitatively analyzed. Therefore, the H2 component becomes the H containing component in claims, and the CO component and the N₂ component become the components of the other elements in the claims.

First, while the He gas is introduced into the heating furnace 10, the crucible 11 into which no sample is input is heated by the heating furnace 10. Then, after the He gas derived from the heating furnace 10 passes through the dust filter 70 and the oxidation part 80, the He gas is introduced into the sensor part 41 of the quadrupole mass spectrometer 40. As this result, the reference data preparing part 42a prepares the reference data D1 wherein the chronological change of the signal intensity of the He gas introduced into the sensor part 41 is plotted (a reference data preparation process).

Next, the sample is put into the crucible 11 of the heating furnace10. Then, while the He gas is introduced into the heating furnace 10, the crucible 11 in which the sample is fed is heated by the heating furnace 10. Then, the sample gas containing the CO component, the N2 component and the H2 component is generated in the heating furnace 10. Then, after the mixed gas derived from the heating furnace 10 passes through the dust filter 70 and the oxidation part 80, the mixed gas is introduced into the sensor part 41 of the quadrupole mass spectrometer 40.

Here, the components contained in the sample gas in the mixed gas derived from the heating furnace 10 are introduced into the sensor part 41 of the quadrupole mass spectrometer 40 in a state of being oxidized by the oxidation part 80. Concretely, among the CO component, the N2 component and the H2 component contained in the sample gas in the mixed gas, the CO component and the H2 component contained in the sample gas in the mixed gas are introduced into the sensor part 41 of the quadrupole mass spectrometer 40 as the CO₂ component and the H₂O component in an oxidized state by the oxidation part 80. Therefore, the H2 component is changed into the H₂O component having a higher mass number than that of the H2 component by passing through the oxidation part 80, and the CO component having the same mass number as that of the N2 component passes through the oxidation part 80 and is changed into the CO₂ component having a different mass number than that of the N2 component. In other words, some component contained in the sample gas in the mixed gas is changed into a component whose mass number is bigger by being oxidized by the oxidation part 80, and one of the two other element containing components, having the same mass number contained in the sample gas in the mixed gas, is changed into the other element containing component whose mass number is different due to being oxidized by the oxidation part 80 (concretely, the two other element containing components wherein the a mass number difference between the two other elements of the two other element containing components is three or less). That is, the mixed gas containing the CO2 component, the N2 component and the H₂O component will be introduced into the sensor part 41 of the quadrupole mass spectrometer 40.

Then, the measurement data preparation part 42b prepares measurement data D2, which plots the chronological change of the signal intensity for each of the components in the mixed gas introduced into the sensor part 41 (measurement data preparation process).

Since the components contained in the sample gas in the mixture gas derived from the heating furnace 10 change depending on the sample introduced into the heating furnace 10, there might be a case wherein the mixed gas containing each component, for example, the CH4 component (mass number 16), the NH3 component (mass number 17) and the H₂O component (mass number 18) is derived, or a case wherein the mixed gas containing the C₂H₂ component (mass number 26), the HCN component (mass number 27) and the C2H4 component (mass number 28) is derived. Each of the components contained in these mixed gases has a mass number difference of three or less from each other. In spite of these mixed gases, it is possible to increase the mass difference between each of the components contained in the mixed gas introduced into the sensor part 41 of the quadrupole mass spectrometer 40 by oxidizing at least some of the components by passing through the oxidation section 80.

Further, in the quadrupole mass spectrometer 40, the measurement data D2 corresponding to each of the components is sequentially prepared while changing the detected components by changing the voltage applied to a quadrupole of the sensor part 41. In addition, the pressure control part sequentially adjusts the pressure in the chamber 30 according to the components detected by the sensor part 41 to be the set pressure corresponding to the component by means of the pressure adjusting mechanism 50.

Next, the peak time zone specifying part 42c specifies the peak time period (Z) wherein the peak (P) is detected in each measurement data D2 corresponding to the CO2 component, the N₂ component, and the H₂O component.

Next, the correction value calculating part 42d calculates, as the correction value, an area of a part surrounded by the reference data D1 and the straight line 11 connecting the starting point SP1 and the ending point EP1 in the predetermined time zone (Z') that is determined so as not to include the time when the signal intensity in the reference data D1 becomes the maximum value and the minimum value and to include the peak time zone (Z).

Then, the analysis part 42e calculates, for each of the measurement data D, the area of the part surrounded by the measurement datum D2 and the straight line 12 connecting the starting point SP2 and the ending point EP2 in the predetermined time zone (Z') in the measurement data D2, and calculates a concentration of ions of each component by subtracting the correction value from the calculated value of the area. Then, the analysis part 42e calculates the concentration of each component and an amount of the element to be analyzed contained in each of the component based on the ion concentration of each component.

To be more precise, in either the reference data preparation process or the measurement data preparation process, when the crucible 11 is heated by the heating furnace 10, the gas derived from the crucible 11 is also generated. Therefore, the gas derived from the crucible 11 is also included in the gas (the carrier gas and the mixed gas) introduced from the heating furnace 10.

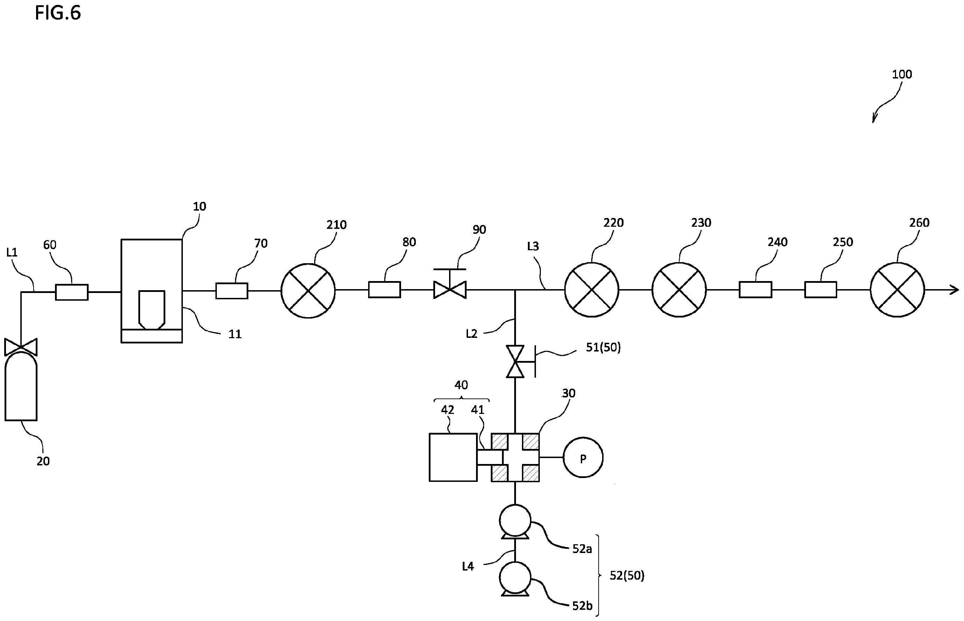

<Other Embodiments> An element analysis device 100 shown in Fig. 6 is represented as another embodiment. The element analysis device 100 shown in Fig. 6 has the same configuration as that of the element analysis device in accordance with the first embodiment except that a first concentration meter 210 is arranged between the dust filter 70 and the oxidation part 80 in the downstream line L2, and a second concentration meter 220, a third concentration meter 230, a de-CO₂ part 240, a de-H₂O part 250 and a TCD 260 are arranged in this order from the upstream side to the downstream side in the first exhaust line L3.

The first concentration meter 210, the second concentration meter 220, and the third concentration meter 230 are so-called NDIRs, which are concentration meters using a non-dispersive infrared absorption method. Concretely, the first concentration meter 210 is used to detect the CO component having a relatively high concentration contained in the sample gas in the mixed gas. In addition, the second concentration meter 220 is used to detect the CO2 component having a relatively high concentration contained in the sample gas in the mixed gas. Furthermore, the third concentration meter 230 is used to detect the H₂O component having a relatively high concentration contained in the sample gas in the mixed gas.

The de-CO₂ part 240 is to remove the CO2 component contained in the mixed gas derived from the oxidation part 80, and the de-H₂O part 250 is to remove the H₂O component contained in the mixed gas derived from the oxidation part 80.

The TCD 260 is a nitrogen detector. The TCD 260 is used to detect the N2 component contained in the mixed gas that is derived from the oxidation part 80 and that passes through the de-CO₂ part 240 and the de-H₂O part.

In accordance with this arrangement, it is possible to conduct the quantitative analysis sequentially on each of the components having a relatively high concentration contained in the mixed gas derived from the heating furnace 10 by means of the first concentration meter 220, the second concentration meter 230, the third concentration meter 240 and the TCD 260. In addition, if the opening of the pressure adjusting valve 51 is adjusted, it is possible for the quadrupole mass spectrometer 40 to conduct the quantitative analysis sequentially on an extremely trace amount of each component contained in the mixed gas. With this arrangement, it is possible to expand a range measured by the device so that the sample gas containing more components can be analyzed.

In addition, it is a matter of course that the present claimed invention is not limited to the above-mentioned embodiment and may be variously modified without departing from a spirit of the invention.

In accordance with the element analyzing device of the present claimed invention, it is possible to quantitatively analyze the H element contained in the sample gas with high accuracy.

## Claims

1. An element analysis device comprising
a heating furnace that heats a crucible into which a sample is contained while introducing a carrier gas to generate a sample gas containing an H element by vaporizing at least a part of the sample and derives the sample gas as a mixed gas as being a mixture of the sample gas and the carrier gas, and
a mass spectrometer that quantitatively analyzes at least one element contained in the sample gas in the mixed gas that is derived from the heating furnace, wherein
the mass spectrometer quantitatively analyzes the H element contained as an H₂O component in the mixed gas regarded as the H element contained in the sample gas.

2. The element analysis device described in claim 1, further comprising
an oxidation part arranged between the heating furnace and the mass spectrometer, wherein
the heating furnace generates the sample gas containing the H element as an H containing component and derives the mixed gas containing the H containing component,
the oxidation part oxidizes the H containing component in the mixed gas to generate the H₂O component, and
the mass spectrometer quantitatively analyzes the H element contained as the H₂O component in the oxidized mixed gas regarded as the H element contained in the sample gas.

3. The element analysis device described in claim 2, wherein
the heating furnace generates the sample gas that contains the H element as the H containing component and that contains a plurality of elements other than the H element as other element containing components wherein each element differs, and derives the mixed gas containing the H containing component and the plurality of the other element containing components,
the oxidation part generates the H₂O component by oxidizing the H containing component in the mixed gas and oxidizes at least one of the two other element containing components wherein a mass number difference between the two other elements is three or less among the two other element components in the mixed gas, and
the mass spectrometer quantitatively analyzes the H element contained as the H₂O component in the oxidized mixed gas regarded as the H element contained in the sample gas and quantitatively analyzes the other element other than the H element contained as the other element containing component in the oxidized mixed gas regarded as the other element contained in the sample gas.

4. The element analysis device described in claim 3, wherein
the heating furnace generates the sample gas that contains the H element as the H containing component and that contains an N element and an O element as the other elements regarded as an N2 component and a CO component, and derives the mixed gas that contains the H containing component, an N₂ containing component and a CO containing component, and
the oxidation part generates the H₂O component by oxidizing the H containing component in the mixed gas and generates the CO₂ component by oxidizing the CO component in the mixed gas.

5. The element analysis device described in claim 1, wherein
the carrier gas is a He gas.

6. The element analysis device described in claim 1, wherein
the mass spectrometer is a quadrupole mass spectrometer,
the quadrupole mass spectrometer comprises an operation part,
the operation part comprises
a reference data preparing part that prepares a reference data indicating a chronological change of a signal intensity by applying heat to the crucible by the heating furnace in a state wherein no sample is fed into the crucible while introducing the carrier gas into the heating furnace, and by introducing the carrier gas derived from the heating furnace into the quadrupole mass spectrometer,
a measurement data preparing part that prepares a measurement data indicating the chronological change of the signal intensity by applying heat to the crucible by the heating furnace in a state wherein the sample is fed into the crucible while the carrier gas is introduced into the heating furnace, and by introducing the mixed gas derived from the heating furnace into the quadrupole mass spectrometer,
a peak time zone specifying part that specifies a peak time zone in the measurement data when a peak of the signal intensity corresponding to the component containing the element contained in the sample gas in the mixed gas is detected,
a correction value calculating part that calculates, as a correction value, an area of a part surrounded by the reference data and a straight line connecting a starting point and an ending point in a predetermined time zone that is determined so as not to include time when the signal intensity in the reference data become the maximum value and the minimum value and to include the peak time zone, and
an analyzing part that quantitatively analyzes the element contained in the sample gas based on the measurement data and the correction value.

7. The element analysis device described in claim 1, further comprising
a chamber that is arranged in a downstream side of the heating furnace and to which the mass spectrometer is connected, and
a pressure adjusting mechanism that can adjust pressure in the chamber according to the element to be quantitatively analyzed by the mass spectrometer.

8. The element analysis device described in claim 7, further comprising
a pressure sensor that measures the pressure in the chamber, and
the pressure adjusting mechanism comprises
a pressure control part that controls the pressure value measured by the pressure sensor to approach a predetermined set pressure according to the element to be quantitatively analyzed by the mass spectrometer.

9. An element analysis method to quantitatively analyze an element contained in a sample gas generated by vaporizing a sample, wherein
applying heat to a crucible having the sample contained therein while introducing a carrier gas to generate the sample gas that contains an H element by vaporizing at least a part of the sample, introducing the H element in a mixed gas comprising the sample gas and the carrier gas derived from the heating furnace into a mass spectrometer as an H₂O component, and quantitatively analyzing the H element contained as the H₂O component in the mixed gas by the use of the mass spectrometer.

10. The element analysis method described in claim 9, wherein
preparing a reference data indicating a chronological change of a signal intensity by applying heat to the crucible by the heating furnace in a state wherein no sample is fed into the crucible while the carrier gas is introduced into the heating furnace, and by introducing the carrier gas derived from the heating furnace into the quadrupole mass spectrometer,
preparing a measurement data indicating the chronological change of the signal intensity by applying heat by the heating furnace to the crucible in a state wherein the sample is fed into the crucible while the carrier gas is introduced into the heating furnace, and by introducing the mixed gas derived from the heating furnace into the quadrupole mass spectrometer,
specifying a peak time zone in the measurement data when a peak of the signal intensity corresponding to the component containing the element contained in the sample gas in the mixed gas is detected,
calculating an area of a part surrounded by the reference data and a straight line connecting a starting point and an ending point in a predetermined time zone that is determined so as not to include time when the signal intensity in the reference data becomes the maximum value and the minimum value and so as to include the peak time zone as a correction value, and
quantitatively analyzing the element contained in the sample gas based on the measurement data and the correction value.
